# EUROPEAN PATENT APPLICATION

(11) **EP 1 826 567 A1**
(43) Date of publication of application: **29.08.2007**
(21) Application number: 05816756.0
(22) Date of filing: 14.12.2005
(51) Int. Cl.: G01N 33/574

(54) **DRUG FOR DIAGNOSING COLON CANCER AND/OR COLON POLYP, OBSERVING POSTOPERATIVE COURSE AND MONITORING REOCCURRENCE**

(30) Priority: 16.12.2004 JP 2004364731
(71) Applicant: Perseus Proteomics Inc., Tokyo 153-0041 (JP); The University of Tokyo, Bunkyo-Ku, Tokyo 113-8654 (JP)
(72) Inventor: ABURATANI, Hiroyuki, Musashino-shi, Tokyo 180-0003 (JP); SHIMAMURA, Takahiro, Tokyo 151-0072 (JP); WATANABE, Kiyotaka, Tokyo University Hospital, Tokyo 113-8655 (JP); ASANO, Takeharu, Tokyo University, Tokyo 113-0033 (JP); OHNISHI, Shin, Tokyo 113-0024 (JP); HAMAKUBO, Takao, The University of Tokyo, Tokyo 153-0041 (JP); SUGIYAMA, Akira, Perseus Proteomics Inc., Tokyo 153-0041 (JP); HOSOMI, Naoki, c/o Perseus Proteomics Inc., Tokyo 153-0041 (JP); IWANARI, Hiroko, c/o Perseus Proteomics Inc., Tokyo 153-0041 (JP); ISHII, Keisuke, c/o Perseus Proteomics Inc., Tokyo 153-0041 (JP)
(74) Representative: Hartz, Nikolai
(86) International application number: PCT/JP2005/022955
(87) International publication number: WO 2006/064844

(57) **Abstract**

The present invention is directed to a method for diagnosing large intestinal cancer and/or polyp and a method for observing postoperative course or monitoring recurrence thereof, wherein each method includes detecting cystatin SN protein by use of an anti-cystatin SN antibody. The present invention is able to provide a kit for assaying cystatin SN, which can be used, in a simple manner, in a diagnosis performed prior to conventional barium enema examination and endoscopic examination which impose burdens on patients; as an indicator of metastasis and recurrence; and in the evaluation of therapeutic effects. The present invention provides a method for diagnosing or monitoring large intestinal cancer and/or polyp which can be performed in a simple manner, and thus can allow to design a new regimen rapidly.

## Description

### Technical Field

The present invention relates to a method for diagnosing large intestinal cancer and/or polyp, a method for observing postoperative course or monitoring recurrence thereof, and a drug for the diagnosis, the observation or the monitoring.

### Background Art

In Japan, the number of patients suffering from large intestinal cancer is increasing considerably in connection with the trend of Westernization of the diet. The annual number of the patients is now about 60,000 and is estimated to exceed that of gastric cancer by the year of 2015. The incidence of large intestinal cancer is estimated to be the highest among all cancers in women, and the third highest in men following lung cancer and hepatoma. According to epidemiological studies, large intestinal cancer is thought to be more attributable to the diet, particularly excessive ingestion of animal fat and protein, than to genetic character. Onset of large intestinal cancer is preferentially observed in the sigmoid and the rectum.

There are several methods for detecting large intestinal diseases such as large intestinal cancer and polyps; for example, (i) blood examination, (ii) occult blood test, (iii) barium enema examination, (iv) endoscopic examination, (v) examination by means of PET and an encapsulated endoscopy, and (vi) gene diagnosis. The methods (i) and (ii), which can be performed in a simple manner, are poor in accuracy. Although the methods (iii) and (iv) are widely employed as the most reliable methods, in many cases, these methods not only afflict patients but also raise a problem in terms of medical economy. Thus, similar to the diagnosis of other diseases, there is a demand for development of a simple method for diagnosing large intestinal diseases precisely in their early stages without causing pain to the patients.

Large intestinal polyp or cancer may be detected by conducting various examinations on patients who complain abnormality in the abdomen. In many cases where patients are first diagnosed as having large intestinal polyps, if no abnomal cell proliferation is observed in a follow-up observation, the polyps are generally diagnosed as a benign tumor. However, some patients exhibit abnomal cell proliferation of the polyp in the follow-up observation, and are diagnosed with large intestinal cancer. In the latter case, the patients are supposed to have been in a precancerous state rather than polyp. If the determination whether the tumor is benign or malignant is possible with a simple method not in the follow-up observation but in an initial diagnosis, not only the improvement of QOL of the patients but also the reduction of medical expense can be attained.

When detected in an early stage, large intestinal cancer can be completely cured through endoscopic resection or a surgical therapy. However, if detection is delayed, distant metastasis to the liver or the lungs may often occur, and probability of postoperative recurrence may increase. Therefore, early detection of the cancer and examination after resection of the cancer are important, and there is a demand for a simple method for diagnosing the cancer precisely without causing pain to the patients.

Cystatin SN is a species of cysteine proteinase inhibitors existing in the living body. Cysteine proteinase inhibitors are categorized into three families (Stefins, Cystatins, and Kinogens). Cystatins include cystatin S, cystatin SA, and cystatin SN, which are present in saliva, tears, and semen. Cystatins play an important role in preventing proteins from pathologic decomposition. The cystatin SN gene and protein have already been elucidated by Saitoh E. and others (Non-Patent Documents 1, 2, and 3), and preparation of an antiserum against cystatin SN is also disclosed in Non-Patent Document 3.

Since cystatin SN is contained abundantly in saliva and tears, however, diseases related to cystatin SN have been studied only in relation to a periodontal disease (Patent Document 1) and migraine headache (Patent Document 2).
Patent Document 1: WO 2001/79297
Patent Document 2: WO 2001/79270
Non-Patent document 1: FEBS Lett., 1986, Mar 17; 198(1)145-9
Non-Patent document 2: Gene. 1987; 61(3): 329-38
Non-Patent document 3: J. Biochem. (Tokyo), 1991 Oct; 110(4): 648-54

### Disclosure of the Invention

### Problems to be Solved by the Invention

Under such circumstances, the present inventors previously found that expression of a cystatin SN gene was promoted in large intestinal cancer, which was observed through gene chip analysis of various cancers; and that such promotion of the gene expression in the large intestinal cancer tissue was also confirmed through RT-PCR; and that promotion of the protein level in an extract of the tissue was confirmed through Western blotting analysis (Japanese Patent Application No. 2003-290704).
Although the studies conducted by the present inventors have elucidated promotion of the cystatin SN gene level and cystatin SN protein level in large intestinal cancer tissues, clinical use of cystatin SN as a diagnostic drug for applying to tissue samples has a lot of problems. Specifically, the problems are accompanied by excision of tissues which is a burden to patients; cumbersome operations such as extraction of cystatin SN from the tissues and electrophoresis; and increased medical expense.

Thus, an object of the present invention is to provide a means for diagnosing large intestinal cancer and polyp, and a means for observing postoperative course or monitoring recurrence thereof, wherein the means employ body fluid specimens such as blood so as not to impose burdens on patients.

### Means for Solving the Problems

The present inventors have developed a novel antibody against cystatin SN and determined the cystatin SN level in the serum of large intestinal cancer patients instead of the cystatin SN level in large intestinal cancer tissues. As a result, 48 healthy subjects exhibited a serum cystatin SN level of 0.000 to 2.303, with an average of 0.794, whereas large intestinal cancer patients exhibited a serum cystatin SN level of 0.000 to 36.393, with an average of 1.911. Furthermore, time-lapse monitoring of a serum sample after excision of large intestinal cancer has revealed that the serum cystatin SN level was reduced through the excision of the cancer, and that the cystatin SN level was increased through the recurrence of the cancer. In addition, the inventors have successfully enhanced the sensitivity of the assay system to enable to diagnose large intestinal polyp with the system. The present invention has been accomplished on the basis of these findings.

Accordingly, the present invention provides a drug for diagnosing large intestinal cancer and/or polyp, and a drug for observing postoperative course or monitoring recurrence thereof, wherein each of the drugs contains an anti-cystatin SN antibody.
The present invention also provides a method for diagnosing large intestinal cancer and/or polyps, and a method for observing the postoperative course or monitoring recurrence thereof, wherein each of the methods comprises determining cystatin SN level in a test sample by use of an anti-cystatin SN antibody.

### Effects of the Invention

The present invention is able to provide a kit for assaying cystatin SN, which can be used, in a simple manner, in the diagnosis performed prior to conventional barium enema examination and endoscopic examination which impose burdens on the patients; as an indicator of metastasis and recurrence; and in the evaluation of therapeutic effects. Thus, the present invention provides a simple method of diagnosis and monitoring, and can allow to design a new regimen rapidly.

### Brief Description of the Drawings

[Fig. 1] Detection of cystatin SN in supernatants from various cancer cell cultures through Western blotting.
[Fig. 2] Calibration curve for determining cystatin SN level obtained from an ELISA system.
[Fig. 3] Distribution profile of cystatin SN levels of healthy subjects and large intestinal cancer patients.
[Fig. 4] Change over time in cystatin SN level after excision of large intestinal cancer (1)
[Fig. 5] Change over time in cystatin SN level after excision of large intestinal cancer (2)
[Fig. 6] Comparison in sensitivity between the ELISA system employed in the present invention and the conventional ELISA system.
[Fig. 7] Correlation of assay results between the ELISA system employed in the present invention and the conventional ELISA system.
[Fig. 8] Distribution profile of serum cystatin SN level of large intestinal disease patients determined through the assay system of the present invention.

### Best Modes for Carrying Out the Invention

The present invention is described hereinafter in detail.
The present invention is directed to a method for diagnosing large intestinal cancer and/or polyps and to a method for observing postoperative course or monitoring recurrence thereof, wherein each of the methods comprises determining cystatin SN level of a sample by use of an anti-cystatin SN antibody. Cystatin SN is a proteinase inhibitor, and the amino acid sequence thereof and the nucleotide sequence encoding the amino acid sequence are disclosed in GenBank No. (NM_001898) (SEQ ID NOs: 1 and 2). In the present invention, the term "cystatin SN protein" means the full-length protein and a fragment thereof. The term "fragment" means a polypeptide including any region of cystatin SN protein, and may not have a function as a native cystatin SN protein exhibits.

The cystatin SN protein detected in the present invention is preferably human cystatin SN protein. However, any other cystatin SN proteins may also be detected, such as dog cystatin SN, cat cystatin SN, mouse cystatin SN, and hamster cystatin SN.

In the present invention, the term "detection" means both quantitative and non-quantitative detection. Examples of non-quantitative detection include a measurement to simply check the presence of cystatin SN protein, a measurement to determine whether cystatin SN protein of a predetermined amount or more is present or not, and a measurement to determine the relative amount of cystatin SN protein in a sample with respect to those in other samples (e.g., a control sample). Examples of the quantitative detection include a determination of cystatin SN protein level and a determination of the amount of cystatin SN protein.

No particular limitation is imposed on the type of the assay sample, so long as the sample may contain cystatin SN protein. Preferably, the sample is collected from an organism such as a mammal, more preferably from a human. Specific examples of the assay sample include blood, interstitial fluid, plasma, extravascular fluid, cerebrospinal fluid, synovial fluid, pleural fluid, serum, lymph, saliva, and urine. Of these, blood, serum, and plasma are preferred. Further, assay samples such as solution from cell culture derived from an organism body also fall within the scope of the assay sample of the present invention.

The disease diagnosed by the invention is large intestinal cancer and/or polyp.

In the present invention, when cystatin SN protein is detected in a specimen, the cystatin SN protein level is determined. When the cystatin SN protein level is found to be higher as compared with that of a negative control or a healthy subject, the patient is diagnosed with large intestinal cancer and/or polyp, or to have higher possibility of developing large intestinal cancer in future. Furthermore, when the cystatin SN protein levels determined after excision of large intestinal cancer tissues or chemotherapy are reduced compared with the level before the surgery or the therapy, the postoperative course and the post-chemotherapy course are determined to be in good conditions. In contrast, when the cystatin SN protein levels determined after the surgery or the chemotherapy are elevated, the patient is determined to have recurrence or metastasis.

Cystatin SN protein contained in an assay sample is detected through immunoassay by use of an anti-cystatin SN antibody. Examples of immunoassay include radioimmunoassay, enzyme immunoassay, fluoroimmunoassay, luminescent immunoassay, immunoprecipitation, immunonephelometry, Western blotting, immunostaining, and immunodiffusion. Of these, enzyme immunoassay is preferred, with enzyme-linked immunosorbent assay (ELISA) (e.g., sandwich ELISA) being particularly preferred. The aforementioned immunoassay methods including ELISA may be carried out through a technique known in the art.

Example of commonly-used detection method employing an anti-cystatin SN antibody includes a method comprising immobilizing an anti-cystatin SN antibody onto a support, adding an assay sample thereto, incubating them to bind the anti-cystatin SN antibody with cystatin SN protein, followed by washing, and detecting cystatin SN protein bound to the support using an anti-cystatin SN antibody, thereby detecting cystatin SN protein contained in the assay sample.

Examples of the support employed for immobilization of the anti-cystatin SN antibody in the present invention include supports made from insoluble polysaccharide such as agarose or cellulose; synthetic resin such as silicone resin, polystyrene resin, polyacrylamide resin, nylon resin or polycarbonate resin; or insoluble material such as glass. These supports may be used in the form of beads or a plate. In the case of beads, a column or the like which filled with the beads may be employed. In the case of plate, a multi-well plate (e.g., 96-well multi-well plate), a biosensor chip or the like may be employed. Immobilization of the anti-cystatin SN antibody on the support may be carried out through a conventional method such as chemical binding or physical adsorption. All of these supports used in the invention can be commercially available.

Generally, binding of cystatin SN protein to the anti-cystatin SN antibody is performed in a buffer. Examples of the buffer include phosphate buffer, Tris buffer, citrate buffer, borate buffer, and carbonate buffer. Incubation is performed under generally employed conditions; for example, 4°C to 37°C for one hour to 24 hours. After incubation, the support is washed. No particular limitation is imposed on the washing solution, so long as it does not impede binding of cystatin SN protein to the anti-cystatin SN antibody. For example, a buffer containing a surfactant such as Tween 20 is employed.

In addition to an assay sample to be detected cystatin SN protein therein, a control sample may be used in the method of detecting cystatin SN protein of the present invention. Examples of the control sample include a negative control sample containing no cystatin SN protein and a positive control sample containing cystatin SN protein. In the case where control samples are employed, cystatin SN protein present in the assay sample can be detected by comparing the results from the assay sample with those from the negative control sample containing no cystatin SN protein, or with those from the positive control sample containing cystatin SN protein. In an alternative method, a series of control samples having stepwise-graded concentrations are prepared. A standard curve is obtained from the detection data from each of the control samples. With reference to the standard curve, cystatin SN protein contained in an assay sample can be detected quantitatively.

In one preferred embodiment, cystatin SN protein bound to the support via an anti-cystatin SN antibody is detected by use of an anti-cystatin SN antibody labeled with a labeling substance. For example, cystatin SN protein is detected by contacting an assay sample with the anti-cystatin SN antibody immobilized on the support, followed by washing, and using the labeled antibody which specifically recognizes cystatin SN protein.

Labeling of the anti-cystatin SN antibody may be performed through a commonly known method. Labeling substances known to those skilled in the art, such as a fluorescence dye, an enzyme, a co-enzyme, a chemoluminescence substance and a radioactive substance, may be employed. Specific examples include radioisotopes (e.g., ³²P, ¹⁴C, ¹²⁵I, ³H, and ¹³¹I), fluorescein, rhodamine, dansyl chloride, umbelliferone, luciferase, peroxidase, alkaline phosphatase, β-galactosidase, β-glucosidase, horseradish peroxidase, glucoamylase, lysozyme, saccharide oxidase, microperoxidase, and biotin. When biotin is used as a labeling substance, streptoavidin bound to an enzyme (e.g., peroxidase) is preferably added after addition of biotin-labeled antibody. Binding of the labeling substance to the anti-cystatin SN antibody may be performed through a known method such as the glutaraldehyde method, the maleimide method, the pyridyldisulfide method, or the periodate method.

In a specific procedure, a solution containing an anti-cystatin SN antibody is added to a support such as a plate, thereby immobilizing the anti-cystatin SN antibody on the support. After washing of the plate, blocking is performed by use of BSA, gelatin, albumin, etc. in order to prevent non-specific binding of protein. The plate is washed again, and an assay sample is added to the plate. After incubation, the plate is washed, and a labeled anti-cystatin SN antibody is added. After incubating appropriately, the plate is washed again, and a labeled anti-cystatin SN antibody remaining on the plate is detected. Detection may be performed through a method known in the art. For example, in the case in which the labeling substance is a radioactive substance, the antibody can be detected through liquid scintillation or the RIA method. When the labeling substance is an enzyme, a substrate is added, and enzymatic change in the substrate (e. g., coloring), can be detected by means of an spectrophotometer. Examples of the substrate include diammonium 2,2-azinobis(3-ethylbenzothiazoline-6-sulfonate) (ABTS), 1,2-phenylenediamine(o-phenylenediamine), and 3,3',5,5'-tetramethylbenzidine (TMB). When the labeling substance is a fluorescence substance, the antibody can be detected by means of a fluorophotometer.

One preferred embodiment of the method of detecting cystatin SN protein of the present invention includes a method employing a biotin-labeled anti-cystatin SN antibody and streptoavidin.

In a specific procedure, a solution containing an anti-cystatin SN antibody is added to a support such as a plate, thereby immobilizing the anti-cystatin SN antibody on the support. After washing the plate, blocking is performed by use of BSA or the like to prevent non-specific binding of protein. The plate is washed again, and an assay sample is added to the plate. After incubation, the plate is washed, and a biotin-labeled anti-cystatin SN antibody is added. After incubating appropriately, the plate is washed again, and an avidin bound to an enzyme such as alkaline phosphatase or peroxidase is added. After incubation, the plate is washed, and a specific substrate of the enzyme bound to the avidin is added. Cystatin SN protein is detected based on an indicator such as enzymatic change of the substrate.

Another embodiment of the method of detecting cystatin SN protein of the present invention includes a method employing at least one primary antibody which specifically recognizes cystatin SN protein, and at least one secondary antibody which specifically recognizes the primary antibody.

In an exemplified procedure, an assay sample is brought into contact with at least one anti-cystatin SN antibody immobilized on the support. After incubation, the support is washed. Cystatin SN protein remaining after washing is detected by a primary anti-cystatin SN antibody and at least one secondary antibody which specifically recognize the primary antibody. In this case, the secondary antibody is preferably labeled with a labeling substance.

Still another embodiment of the method of detecting cystatin SN protein of the present invention includes a detection method employing coagulation. In the method, cystatin SN protein can be detected by a support sensitized by an anti-cystatin SN antibody. No particular limitation is imposed on the type of the support which is sensitized by the antibody, and any support may be employed, so long as the support is insoluble, causes no non-specific reaction, and is stable. For example, latex particles, bentonite, collodion, kaolin, fixed sheep erythrocytes, etc. may be employed. Of these, latex particles are preferably employed. Examples of employable latex particles include polystyrene latex particles, styrene-butadiene copolymer latex particles, and polyvinyltoluene latex particles. Of these, polystyrene latex particles are preferably employed. Sensitized particles and a sample are mixed together, and the mixture is stirred for a predetermined period of time. When the amount of cystatin SN contained in the sample increases, the extent of particle coagulation increases. Thus, cystatin SN protein can be detected through visual observation of coagulates. Alternatively, turbidity caused by coagulation is measured to detect cystatin SN protein by means of a spectrophotometer or the like.

Yet another embodiment of the method of detecting cystatin SN protein of the present invention includes a method employing a biosensor on the basis of surface plasmon resonance. The biosensor employing surface plasmon resonance enables real-time observation of protein-protein interaction as a surface plasmon resonance signal with a small amount of proteins and without labeling them. For example, using a biosensor such as BIA core (Biacore International AB), binding between a cystatin SN protein and an anti-cystatin SN antibody can be detected. Specifically, an assay sample is brought into contact with a sensor chip on which an anti-cystatin SN antibody has been immobilized, whereby cystatin SN protein bound to the anti-cystatin SN antibody can be detected as change in a resonance signal.

The detection method of the present invention may be automated by use of a variety of automated inspection apparatuses. Thus, a large number of samples may be assayed at one time.

An object of the present invention is to provide a diagnostic drug or a kit for detecting cystatin SN protein present in an assay sample in order to diagnose large intestinal cancer and/or polyps. The diagnostic drug or the kit essentially contains an anti-cystatin SN antibody. When the diagnostic drug or the kit is designed on the basis of EIA methods such as ELISA method, the drug or the kit may contain a support for immobilizing the antibody. Alternatively, the antibody may be bound to the support in advance. When the diagnostic drug or the kit is used on the basis of the coagulation method employing a support made of latex or the like, the drug may contain a carrier onto which the antibody is adsorbed. In addition, the kit may optionally contain a blocking solution, reaction solution, reaction-terminating liquid, reagents for treating samples, or the like.

### Preparation of an anti-cystatin SN antibody

No particular limitation is imposed on the origin, type (monoclonal, polyclonal), and form of the anti-cystatin SN antibody employed in the present invention, so long as it is specifically bound to cystatin SN protein. Specifically, known antibodies such as a mouse antibody, a rat antibody, an avian antibody, a human antibody, a chimera antibody, and a humanized antibody may be employed. Although these antibodies may be polyclonal, monoclonal antibodies are preferred.

Although the anti-cystatin SN antibody immobilized on a support and the anti-cystatin SN antibody labeled with a labeling substance may recognize the same epitope of the cystatin SN molecule, these antibodies preferably recognize different epitopes. The recognition site is not particularly limited.

The anti-cystatin SN antibody employed in the present invention may be obtained as a polyclonal or monoclonal antibody through any known means. The anti-cystatin SN antibody employed in the present invention is preferably a monoclonal antibody derived from mammal or avian. Particularly preferred is a monoclonal antibody derived from mammal. The monoclonal antibody derived from mammal includes that produced by a hybridoma and that produced by a host genetically transformed by an expression vector containing the antibody gene.

Generally, the hybridoma producing a monoclonal antibody may be produced in the following procedure through any known techniques. Specifically, cystatin SN protein is employed as a immunogen, and immunization is performed by use of the antigen through a conventional method. The immunocyte thus obtained is fused with a known parent cell through a conventional cell fusion method. Through a conventional screening method, cells producing a monoclonal antibody can be screened and prepared.
Specifically, the monoclonal antibody may be produced in the following procedure.

Firstly, a cystatin SN protein serving as a immunogen for producing the corresponding antibody is prepared by expressing a cystatin SN gene/amino acid sequence disclosed in GenBank No. (NM_001898). Specifically, a nucleotide sequence encoding cystatin SN is inserted into a known expression vector to transform appropriate host cells. The human cystatin SN protein of interest is purified from the host cells or culture supernatants thereof through a known method. Alternatively, naturally occurring cystatin SN protein may also be purified and employed.

The purified cystatin SN protein is employed as a immunogen. Alternatively, a partial peptide of the cystatin SN protein may also be employed as a immunogen. In the latter case, the partial peptide may be prepared through chemical synthesis based on an amino acid sequence of human cystatin SN protein; may be prepared by inserting a part of the cystatin SN gene into an expression vector; or may be prepared by degrading naturally occurring cystatin SN protein with protease. No particular limitation is imposed on the region and size of the cystatin SN used for preparing the partial peptide.

No particular limitation is imposed on the mammal to be immunized by the immunogen, and the mammal is preferably selected in consideration of adaptability to the parent cell employed in cell fusion. Generally, rodents (e.g., mice, rats, and hamsters), chiken, rabbits, and monkeys are employed.

Immunization of an animal with a immunogen is performed through a known method. For example, in a commonly employed method, the immunogen is injected to a mammal intraperitoneally or subcutaneously. More specifically, a immunogen is diluted with and suspended in an appropriate amount of PBS (Phosphate-Buffered Saline), physiological saline or the like, and if required, an appropriate amount of a conventional adjuvant (e.g., Freund's complete adjuvant) is added to the suspension. The mixture is emulsified and administered to the mammal several times every 4 to 21 days. Upon immunization with a immunogen, an appropriate carrier may also be used. Particularly when a partial peptide having a small molecular weight is employed as a sensitized antigen, the partial peptide is preferably bound to a carrier protein such as albumin or keyhole lympet hemocyanin, and used in immunization.

Once the mammal is immunized and the serum antibody level is determined to be elevated to the desired level, immunocytes are collected from the mammal, and subjected to cell fusion. Particularly preferred immunocytes include spleen cells.

The parent cells to be fused with the aforementioned immunocytes are mammalian myeloma cells. Examples of the myeloma cells preferably employed are various known cell strains such as P3 (P3x63Ag8.653) (J. Immnol. (1979) 123, 1548-1550), P3x63Ag8U.1 (Current Topics in Microbiology and Immunology (1978) 81, 1-7), NS-1 (Kohler. G. and Milstein, C. Eur. J. Immunol. (1976) 6, 511-519), MPC-11 ( Margulies. D. H. et al., Cell (1976) 8, 405-415), SP2/0 (Shulman, M. et al., Nature (1978) 276, 269-270), FO (de St. Groth, S. F. et al., J. Immunol. Methods (1980) 35, 1-21), S194 (Trowbridge, I. S. J. Exp. Med. (1978) 148, 313-323), and R210 (Galfre, G. et al., Nature (1979) 277, 131-133).

Basically, cell fusion between the aforementioned immunocytes and the myeloma cells may be performed according to a known method; for example, a method of Kohler and Milstein (Kohler. G. and Milstein, C., disclosed in Methods Enzymol. (1981) 73, 3-46).

More specifically, in one procedure, the cell fusion is performed in a conventional nutritive culture solution in the presence of a cell fusion promoter. Examples of the fusion promoter employed include polyethylene glycol (PEG) and Sendai virus (HVJ). In addition, in order to enhance fusion efficiency, a fusion aid such as dimethyl sulfoxide may optionally be added.

The ratio of immunocytes to myeloma cells may be predetermined arbitrarily. For example, immunocytes are preferably used in an amount of 1 to 10 times of myeloma cells. Examples of the culture solution employable in the cell fusion include RPMI1640 culture solution and MEM culture solution, which are suitable for proliferation of the myeloma cells, and other conventional culture solutions employed in such type of cell culture. In addition, serum supplements such as fetal calf serum (FCS) may be used in combination.

In the cell fusion, predetermined amounts of the immunocytes and the myeloma cells are sufficiently mixed together in the culture solution, added a PEG solution (e.g., average molecular weight of about 1,000 to 6,000) which is generally in the concentration of 30-60%(w/v) and heated to about 37°C in advance, and the mixture was mixed to form hybridomas of interest. Subsequently, an appropriate culture solution is successively added to the culture and the supernatant of the culture is removed through centrifugation. Such addition and removal are repeated to remove a cell fusion agent or other substances unpreferred for breeding the hybridomas.

The thus-obtained hybridomas are selected through culturing in a conventional selective culture solution (e.g., HAT culture solution (culture solution containing hypoxanthine, aminopterin, and thymidine)). The culturing in the HAT culture solution is continued for a period of time sufficient to kill cells other than target hybridomas (non-fused cells), generally for several days to several weeks. Subsequently, screening and mono-cloning of hybridomas producing the antibody of interest is performed through a conventional limiting dilution method.

Alternatively, the antibody recognizing cystatin SN may be prepared through a method disclosed in WO03/104453.

Screening and mono-cloning of the antibody of interest may be performed through a known screening method based on antigen-antibody reaction. For example, an antigen is immobilized on a support such as polystyrene beads or a commercially available 96-well microtiter plate, and a culture supernatant of the hybridomas is caused to react with the antigen. After washing the support, the antigen is reacted with an enzyme-labeled secondary antibody or the like. Through this procedure, presence of an antibody which reacts with the immunogen in the culture supernatant can be detected. The hybridomas producing the antibody of interest can be cloned through the limiting dilution method or the like, in which the antigen to be used may be that has been employed in immunization.

Instead of the method for producing the hybridoma through immunization of an animal other than human with an antigen, a human antibody of interest which exhibits binding activity to cystatin SN may be obtained through another method (see Japanese Patent Publication (kokoku) No. 1-59878). In the method, human lymphocytes are sensitized in vitro with cystatin SN, and sensitized lymphocytes are fused with human myeloma cells having permanent cell division potential. Still alternatively, cystatin SN (antigen) is administered to a transgenic animal having the complete repertory of human antibody genes in order to collect cells producing the anti-cystatin SN antibody. The cells are immortalized and human antibody against cystatin SN is obtained (see International Patent Application Laid-Open Nos. WO94/25585, WO93/12227, WO92/03918, and WO94/02602).

The thus-produced hybridomas producing a monoclonal antibody can be subcultured in a conventional culture solution, or can be stored in liquid nitrogen for a long period of time.

To prepare the monoclonal antibody from the hybridomas, the hybridomas may be cultured through a conventional method to collect the antibody from the culture supernatant. Alternatively, the hybridomas may be administered to a mammal adaptable to the hybridomas and proliferated therein to collect the antibody from the ascites. The former method is suitable for preparing the monoclonal antibody in high-purity, whereas the latter method is suitable for large-scale preparation of the monoclonal antibody.

In the present invention, there may be employed a recombinant monoclonal antibody which has been produced through a recombination technique, including cloning an antibody gene from hybridomas, integrating the gene into an appropriate vector, and introducing the vector into a host (see, for example, Vandamme, A. M. et al., Eur. J. Biochem. (1990) 192, 767-775, 1990).
Specifically, mRNA coding a variable (V) region of an anti-cystatin SN antibody is isolated from the hybridomas producing the anti-cystatin SN antibody. Isolation of mRNA may be performed through a known method. For example, the total RNA is prepared through a method such as the guanidine ultracentrifugation method (Chirgwin, J. M. et al., Biochemistry (1979) 18, 5294-5299) or the AGPC method (Chomczynski, P. et al., Anal. Biochem. (1987) 162, 156-159), and mRNA of interest is prepared by means of mRNA Purification Kit (Pharmacia) or the like. Alternatively, mRNA can be directly prepared by use of a QuickPrep mRNA Purification Kit (Pharmacia).

From the mRNA thus-produced, cDNA of the antibody V region is synthesized by use of reverse transcriptase. Synthesis of cDNA is performed by use of AMV Reverse Transcriptase First-strand cDNA Synthesis Kit (Seikagaku Corporation) or the like. Synthesis and amplification of cDNA may be performed with 5'-RACE method (Frohman, M. A. et al., Proc. Natl. Acad. Sci. USA (1988) 85, 8998-9002, Belyavsky, A. et al., Nucleic Acids Res. (1989) 17, 2919-2932) by use of 5'-Ampli FINDER RACE Kit (Clontech) and PCR, or the like.

The DNA fragment of interest is purified from the PCR product thus-obtained, and the fragment is ligated to the vector DNA to form recombinant vectors. The recombinant vectors are introduced into *E. coli.* or other hosts. The desired recombinant vectors are prepared by selecting colonies of the host cells. The nucleotide sequence of the DNA of interest is identified through a known method; for example, the dideoxynucleotide chain termination method.

After production of a DNA coding the V region of the anti-cystatin SN antibody of interest, the DNA is introduced into an expression vector containing a DNA coding a desired antibody constant region (C region).

In the production of the anti-cystatin SN antibody employed in the present invention, an antibody gene is introduced into an expression vector such that the antibody is expressed under regulation by expression regulating regions (e.g., enhancer or promoter). Then, the host cells are transformed by the expression vector, to thereby express the antibody.

Expression of the antibody gene may be performed by simultaneously transforming the host cell with separate expression vectors, each integrating a DNA coding an antibody heavy chain (H chain) and a DNA coding an antibody light chain (L chain). Alternatively, a DNA coding both the H chain and the L chain is integrated into a single expression vector to transform the host cells (see WO94/11523).

In addition to the aforementioned host cells, a transgenic animal may be employed to produce a recombinant antibody. For example, an antibody gene is inserted in a gene coding a protein intrinsically produced in milk (e.g., goat β-casein), to thereby prepare it as a fused gene. A DNA fragment including the fused gene into which the antibody gene has been inserted is injected into a goat embryo, and the embryo is introduced into a female goat. As a result, a transgenic goat born from the goat which has received the embryo, or the offspring thereof produces milk from which the desired antibody is obtained. In order to increase the amount of milk containing the desired antibody produced by the transgenic goat, a hormone may be appropriately introduced to the goat (Ebert, K. M. et al., Bio/Technology (1994) 12, 699-702).

In the present invention, in addition to the aforementioned antibody, an artificially modified recombinant antibody (e.g., a chimera antibody or a humanized antibody) may also be used. These modified antibodies may be produced through a known method.

The chimera antibody is produced by ligating the above-obtained DNA coding the antibody V region to the DNA coding the human antibody C region, integrating the ligated DNA into an expression vector, and introducing the vector into a host. Through the conventional process, the chimera antibody useful for the present invention can be produced.

A humanized antibody, which is also called a reshaped human antibody, is obtained through transferring the CDR (complementarity determining region) of a non-human mammal (e.g., mouse) antibody into the complementarity-determining region of a human antibody. General recombination techniques relating to the above process are known (see European Patent Application Laid-Open No. EP 125023 and WO96/02576).

Specifically, a DNA sequence which has been designed so as to bind the CDR of the mouse antibody and the framework region (FR) of the human antibody is synthesized through PCR employing several oligonucleotides as primers, wherein each oligonucleotide has been designed such that it has overlapping regions with the end regions of both the CDR and the FR (see the methodology disclosed in WO98/13388).

The framework region of the human antibody to be linked via the CDR is selected such that the complementarity-determining regions form a favorable antigen binding site. If necessary, an amino acid residue in the framework region in the antibody variable region may be substituted such that the complementarity-determining region of the reshaped human antibody forms an appropriate antigen binding site ( Sato, K. et al., Cancer Res. (1993) 53, 851-856).

The C region for the chimera antibody or the humanized antibody is derived from a human antibody. For example, an H chain may employ Cγ1 , Cγ2, Cγ3, or Cγ4, and an L chain may employ Cκ or Cλ. In order to stabilize the antibody or the production thereof, the human antibody C region may be modified.

A chimera antibody contains a variable region of a non-human mammalian antibody and a constant region of a human antibody. A humanized antibody contains a complementarity-determining region of a non-human mammalian antibody and a framework region and a C region of a human antibody. Since the humanized antibody has a reduced antigenicity against the human body, it is useful as an active ingredient of a remedy.

The antibody employed in the present invention may be a whole molecule of the antibody. Alternatively, the antibody may be a fragment or a modified form of the antibody, so long as cystatin SN is bound thereto. The antibody also includes a divalent antibody and a monovalent antibody. Examples of antibody fragments include Fab, F(ab')2, Fv, Fab/c containing one Fab and the complete Fc, and single-chain Fv (scFv) in which Fv of the H chain or that of the L chain is linked via an appropriate linker. Specifically, an antibody is treated with an enzyme such as papain or pepsin, to thereby form antibody fragments. Alternatively, a gene coding these antibody fragments is constructed; the gene is introduced to an expression vector; and the vector is expressed in appropriate host cells (see, for example, Co, M. S. et al., J. Immunol. (1994) 152, 2968-2976, Better, M. & Horwitz, A. H. Methods in Enzymology (1989) 178, 476-496, Academic Press, Inc., Plueckthun, A. & Skerra, A. Methods in Enzymology (1989) 178, 476-496, Academic Press, Inc., Lamoyi, E., Methods in Enzymology (1989) 121, 652-663, Rousseaux, J. et al., Methods in Enzymology (1989) 121, 663-669, Bird, R. E. et al., TIBTECH (1991) 9, 132-137).

scFv is obtained by linking the V region of the antibody H chain and the V region of the antibody L chain. In scFv, the V region of the H chain and the V region of the L chain are linked to each other via a linker, preferably a peptide linker (Huston, J. S. et al., Proc. Natl. Acad. Sci. U.S.A. (1988) 85, 5879-5883). In scFv, the V region of the H chain and the V region of the L chain may originate from any of the antibodies described in the present specification. As a peptide linker for linking the V regions, any single strand peptide having, for example 12 to 19 amino acid residues, is employed.

The DNA coding scFv may be produced through the following procedure. Specifically, among DNAs coding the H chain of the aforementioned antibody or the V region of the H chain and DNAs coding the L chain or the V region of the L chain, a DNA fragment coding the total or a desired amino acid sequence is selected as a template. The template is amplified through PCR by use of a primer pair defining the two ends. Subsequently, amplification is further conducted with a DNA coding the peptide linker region and a primer pair defining such that the both ends of the linker-coding DNA are linked with the H chain and the L chain.

Once the DNA coding the scFv is prepared, conventional methods can be employed to prepare an expression vector containing the DNA, as well as a host transformed by the expression vector. Using the host, the scFv can be produced through a routine method.

Similarly, these antibody fragments can be produced by preparing and expressing the gene thereof to produce them in a host. In the present invention, the term "antibody" also includes a fragment of the antibody.

As the modified form of the antibody, the anti-cystatin SN antibody bound to a variety of molecules such as a labeling substance may also be employed. In the present invention, the term "antibody" also includes such modified forms of the antibody. These modified forms may be prepared through chemical modification of the obtained antibody. Notably, the method for modifying antibodies has been well established in the art.

The antibody employed in the present invention may be a bispecific antibody. The bispecific antibody may have antigen-binding sites which recognize different epitopes on cystatin SN molecule, or alternatively recognize cystatin SN in one antigen-binding site and recognizes a substance such as a labeling substance the other antigen-binding site. The bispecific antibody may be produced through binding two antibody HL pairs, or through fusing hybridomas producing different monoclonal antibodies, thereby producing fusion cells producing the bispecific antibody. Alternatively, the bispecific antibody may be produced through a genetic engineering technique.

Expression of the antibody gene thus-prepared may be performed through a known method, whereby the corresponding antibody can be produced. In the case of mammalian cells, expression of the gene may be performed through operatively linking a commonly employed useful promoter, an antibody gene to be expressed, and a poly-A signal located on the 3' downstream side thereof. Examples of the promoter/enhancer include human cytomegalovirus immediate early promoter/enhancer.

Examples of other promoter/enhancers for expressing the antibody employed in the present invention include virus promoter/enhancers such as retrovirus, polyoma virus, adenovirus, simian virus 40 (SV40); and promoter/enhancers derived from mammalian cells such as human elongation factor 1α (HEF1α).

When SV40 promoter/enhancer is employed, gene expression can be readily attained through the method of Mulligan et al. (Nature (1979) 277, 108), whereas when HEF1α promoter/enhancer is employed, gene expression can be readily attained through the method of Mizushima et al. (Nucleic Acids Res. (1990) 18, 5322).

In the case of E. *coli,* expression of the gene may be performed through operatively linking a commonly employed useful promoter, a signal sequence for secreting the antibody, and the antibody gene to be expressed. Examples of the promoter include lacZ promoter and araB promoter. When lacZ promoter is employed, expression can be performed through the method of Ward et al. (Nature (1098) 341, 544-546; FASEB J. (1992) 6, 2422-2427), whereas when araB promoter is employed, expression can be performed through the method of Better et al. (Science (1988) 240, 1041-1043).

As a signal sequence for secreting an antibody, pelB signal sequence (Lei, S. P. et al J. Bacteriol. (1987) 169, 4379) may be used to produce the antibody into periplasmic space of E. *coli.* Subsequently, the antibody produced into the periplasmic space is isolated, and the structure of the antibody is appropriately refolded to use.

As a replication origin, those derived from SV40, polyoma virus, adenovirus, bovin pailloma virus (BPV), etc. may be employed. In order to increase the copying number of the gene in host cell system, the expression vector may contain, as a selection marker, an aminoglycoside transferase (APH) gene, a thymidine kinase (TK) gene, an E. *coli* xthanthine guanine phosphoribosyl transferase (Ecogpt) gene, or a dihydrofolic acid reductase (dhfr) gene.

For the production of the antibody employed in the present invention, any expression system; e.g., a eucaryotic cell system or a procaryotic cell system, may be employed. Examples of the eucaryotic cell include established animal cells such as a mammalian cell system, an insect cell system, a mycotic cell system, and a yeast cell system, and examples of the procaryotic cell include bacterial cell systems such as an E. *coli* cell system.

Preferably, the antibody employed in the present invention is expressed in mammalian cells such as CHO cells, COS cells, myeloma cells, BHK cells, Vero cells, and HeLa cells.

Subsequently, the transformed host cells are cultured in vitro or in vivo, whereby an antibody of interest is produced. Culturing of host cells is performed through a known method. Examples of the culture solutions employed include DMEM, MEM, RPMI 1640, and IMDM. A serum supplement such as fetal calf serum (FCS) may be used in combination.

The antibody thus-expressed and produced is isolated from a cell or a host animal, and may be purified to a homogeneous state. Isolation and purification of the antibody employed in the present invention may be performed by means of an affinity column. Examples of the column using protein A columns include Hyper D, POROS, and Sepharose F.F. (product of Pharmacia). However, no particular limitation is imposed on the isolation/purification method. Any methods commonly employed for isolating and purifying protein may be used. For example, the antibody may be isolated and purified by appropriately selecting or combinating the methods such as chromatography column other than the aforementioned affinity column, filter, ultrafiltration, salting out, and dialysis (Antibodies A Laboratory Manual. Ed Harlow, David Lane, Cold Spring Harbor Laboratory, 1988).

### Examples

The present invention is described hereinafter in more detail by way of example, which should not be construed as limiting the invention thereto.

### Example 1 (Cloning of cystatin SN cDNA)

For producing monoclonal antibodies of cystatin SN, a corresponding antigen was produced. Firstly, cloning of a sequence including the full-length ORF region of cystatin SN was performed. A single-strand cDNA library of the salivary gland expressing cystatin SN was prepared in the same procedure as described above. Through employment of the cDNA library as a template and primers Cystatin SN-f (SEQ ID NO: 3) and Cystatin SN-r (SEQ ID NO: 4) designed from GenBank No. (NM-001898), a full-length ORF gene was isolated through PCR. Cystatin SN-F (Hind) primer (SEQ ID NO: 5) and Cystatin SN-R (His-BamHI) primer (SEQ ID NO: 6) were produced so as to include the full-length ORF gene of the cystatin SN. Through employment of the full-length sequence of the cystatin SN as a template and the primers, a fragment of interest was amplified through PCR, and the fragment was inserted into a phCMV vector (Stratagene). Specifically, after nucleotide sequence analysis through a routine method, the fragment was cut out at HindIII and BamHI sites and inserted into a phCMV vector, to thereby yield a transfer vector phCMV-Cystatin SN-His.

### Example 2 (Preparation of an antigen of cystatin SN)

Transfection was performed according to a protocol of TransIT (TaKaRa). Specifically, CHO cells (1 × 10⁵ cells) were placed on a 6-well dish on the previous day of transfection and cultured overnight. Next day, the expression vector phCMV-Cystatin SN-His (8 µg) and TransIT reagent (16 µL) were mixed with serum-free DMEM (100 µL), and the mixture was incubated at room temperature for 20 minutes. Subsequently, the cultured cells were transfected with the mixture. Alternatively, when FUGENE 6 was employed, transfection was performed according to a protocol of Roche Diagnostics. Specifically, CHO cells (8 × 10⁵ cells) were placed on a 10-cm dish on the previous day of transfection and cultured overnight. Next day, the expression vector phCMV-Cystatin SN-His (8 µg) and FUGENE 6 reagent (16 µL) were mixed with serum-free DMEM (400 µL), and the mixture was incubated at room temperature for 20 minutes. The cultured cells were transfected with the mixture. On the following day of the transfection, cloning was performed through the limiting dilution method employing a selection reagent, G418. The supernatant of the cell culture was collected to determine the expression level of cystatin SN, whereby a constitutive clone expressing cystatin SN in an amount of about 5 to 10 µg/mL was selected.

The selected constitutive clones which constantly expresses cystatin SN was cultured for 48 hours in a serum-free medium CHO-S-SFM-II (Invitrogen) (20 mL) placed in a 150-cm² flask, and the culture supernatant was collected. The supernatant was treated with a TARON His-tag Purification Resin (BD Bioscience) or His Trap HP (Amersham Bioscience), to thereby produce and purify cystatin SN-His Tag fused protein. The purified fused protein was dialyzed against PBS. The dialyzed product was employed as an antigen for immunization and also as a standard substance for establishing an ELISA system employed in the present invention.

### Example 3 (Production of monoclonal antibodies against cystatin SN)

A suspension of Cystatin SN-His (100 µg on the protein basis) in PBS was mixed with a Freund's complete adjuvant. The mixture was intraperitoneally injected to BALB/c mice for initial immunization. In the second immunization, Cystatin SN-His (50 µg on the protein basis) prepared in the same manner as described above and a Freund's incomplete adjuvant were mixed together, and the mixture was intraperitoneally injected to the mice. In final immunization, Cystatin SN-His (50 µg on the protein basis) was intravenously injected to the mice. Spleen cells were prepared from each BALB/c mouse, and the cells were fused with mouse P3U1 cells through a routine method employing polyethylene glycol. Screening was performed through ELISA employing Cystatin SN-His, to thereby produce antibodies which specifically bind to Cystatin SN-His.

Through screening, antibodies PPMX0201 and PPMX0202, which are monoclonal antibodies having high specificity to cystatin SN, were successfully produced. Since PPMX0201 can be employed as an ELISA system, the antibody was deposited as FERM AP-20316 in International Patent Organism Depositary, National Institute of Advanced Industrial Science and technology (Central 6th, 1-1-1 Higashi, Tsukuba City, Ibaraki, 305-8566, Japan) on December 8, 2004.

Meanwhile, there are a variety of proteins analogous to cystatin SN. Among the analogs, cystatin S, cystatin SA, and cystatin C, each having high homology to cystatin SN, are supposed to show cross-reactivity with the anti-cystatin SN. Therefore, cross-reactivity of eight monoclonal antibodies which had already produced and newly produced 20 monoclonal antibodies was investigated. As a result, three of the tested antibodies were found to have weak cross-reactivity with cystatin C. From the remaining antibodies having no cross-reactivity, those suitable for immobilization or labeling were selected. As a result, when PPMX0203 was selected as an immobilized antibody and PPMX0204 was selected as a labeling antibody, a high-sensitivity ELISA system was realized. PPMX0203 and PPMX0204 were deposited as FERM BP-10451 and FERM BP-10452, respectively, in International Patent Organism Depositary, National Institute of Advanced Industrial Science and technology (Central 6th, 1-1-1 Higashi, Tsukuba City, Ibaraki, 305-8566, Japan), on November 17, 2005.

### Example 4 (Production of anti-cystatin SN polyclonal antibodies)

By use of the purified Cystatin SN-His, rabbit polyclonal antibodies were produced through a known method. Specifically, an emulsion of the purified Cystatin SN-His was formed by use of an adjuvant, and the emulsion was subcutaneously administered to rabbits for immunization. The immunization was repeated several times, and an increase in antibody titer was confirmed. Thereafter, blood was collected from the rabbits, and serum was isolated. Through precipitation with ammonium sulfate, polyclonal antibodies were prepared from the serum.

### Example 5 (Detection of cystatin SN in culture solutions of various cancer cell lines through Western blotting)

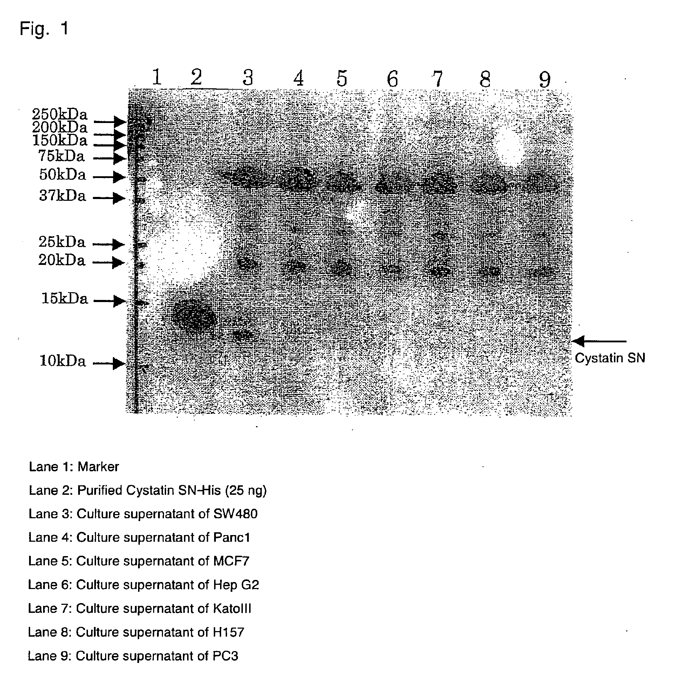

Whether or not cystatin SN is present specifically in large intestinal cancer cell culture solution was investigated by use of culture supernatants of the following cancer cell lines: SW480 (large intestinal cancer cell line), Panc-1 (pancreatic cancer cell line), MCF7 (breast cancer cell line), KatoIII (stomach cancer cell line), Hep G2 (liver cancer cell line), H157 (lung cancer cell line), and PC3 (prostate cancer cell line). Each of the culture supernatants was Western-blotted. Specifically, each cell culture supernatant (8 µL) was applied onto a lane, and the purified Cystatin SN-His was employed as a positive control. SDS-PAGE was performed under reducing conditions. The anti-cystatin SN antibody was reacted with cystatin SN presenting on the membrane. Subsequently, bands attributed to cystatin SN were detected through reaction with an HRP-labeled antimouse IgG antibody. As shown in Fig. 1, a band attributed to cystatin SN was observed in the lane of large intestinal cancer cell line SW480, but no band attributed to cystatin SN was observed in the lanes of cell line other than large intestinal cancer cell line; i.e., Panc-1, MCF7, Hep G2, KatoIII, H157, and PC3.

### Example 6 (Establishment of ELISA system for measuring cystatin SN)

The monoclonal antibodies and polyclonal antibodies against cystatin SN were biotin-labeled with Sulfo-NHS-LC-Biotin (PIERCE). The monoclonal antibodies (10 pg/mL) against cystatin SN were added to Maxi sorp 96-well plate (Nunc) (100 µL/well) and allowed to adsorb on the plates overnight at 4°C. The plate was washed with PBS containing 0.05% Tween-20 (hereinafter referred to as washing solution), and unbound areas of the plate were blocked with an Immunoassay stabilizer (150 µL/well) (ABi biotechnologies) for one hour at room temperature. The plate was washed again with the washing solution, and reacted with purified cystatin SN solutions of various concentrations or a blank solution (each 100 µL/well), at room temperature for one hour. The plate was washed again with the washing solution, and reacted with each of the biotin-labeled monoclonal antibodies and polyclonal antibodies against cystatin SN (1 µg/mL, 100 µL/well) at room temperature for one hour. The plate was washed again with the washing solution, and reacted with streptoavidin-horseradish peroxidase (Vector) (1.0 µg/mL, 100 µL/well), at room temperature for one hour. The plate was washed again with the washing solution, and reacted with a TMB reagent in the dark at room temperature for 30 minutes. Reaction was terminated with a terminating solution. Subsequently, absorbance (at 450 nm) was measured in ELISA plate. On the basis of the results, a combination of a biotin-labeled antibody and an antibody absorbed on a plate which exhibits a signal increase in proportion to the purified cystatin SN concentration and a low blank value was selected. Through further studies on serum sample assay conditions, these antibodies were found to require dilution. Thus, the antibodies were diluted with a 30% normal calf serum (NCS)/PBS/1.0mM EDTA.

### Example 7 (Detection of serum cystatin SN in large intestinal cancer patients and healthy subjects)

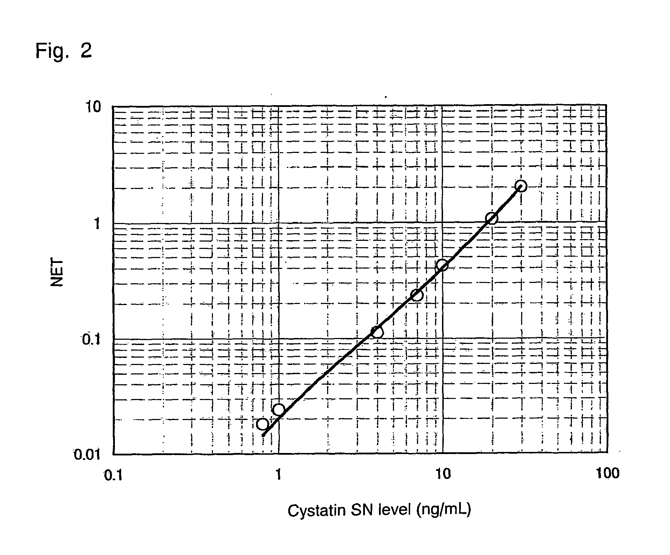

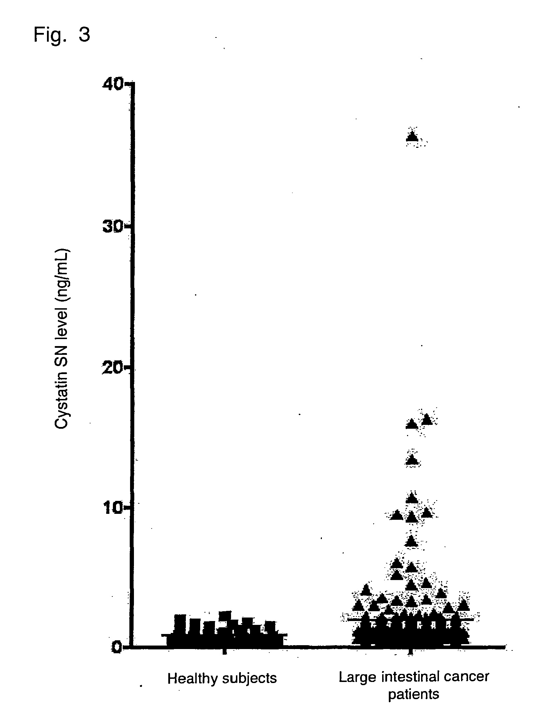

Presence or absence of cystatin SN in serum samples obtained from large intestinal cancer patients was determined through assay of 215 large intestinal cancer patients and 48 healthy subjects. Specifically, the assay was performed through the sandwich ELISA method employing PPMX0201 as an immobilized antibody and a polyclonal antibody produced in Example 4 as a biotin-labeled antibody. A calibration curve (Fig. 2) was made using measured values from the purified cystatin SN of various concentrations and the values obtained by subtracting a blank value from the measured values (NET). Each serum sample was diluted five-fold, and the serum cystatin SN levels were calculated on the basis of the calibration curve. The distribution of the cystatin SN levels is shown in Fig. 3. The data shown in Fig. 3 revealed that 48 healthy subjects exhibited a serum cystatin SN level of 0.000 to 2.303, with an average of 0.794, whereas large intestinal cancer patients exhibited a serum cystatin SN level of 0.000 to 36.393, with an average of 1.911. Therefore, large intestinal cancer can be diagnosed on the basis of serum cystatin SN level determined through ELISA.

### Example 8 (Monitoring of serum cystatin SN in cancer patients after excision of cancer)

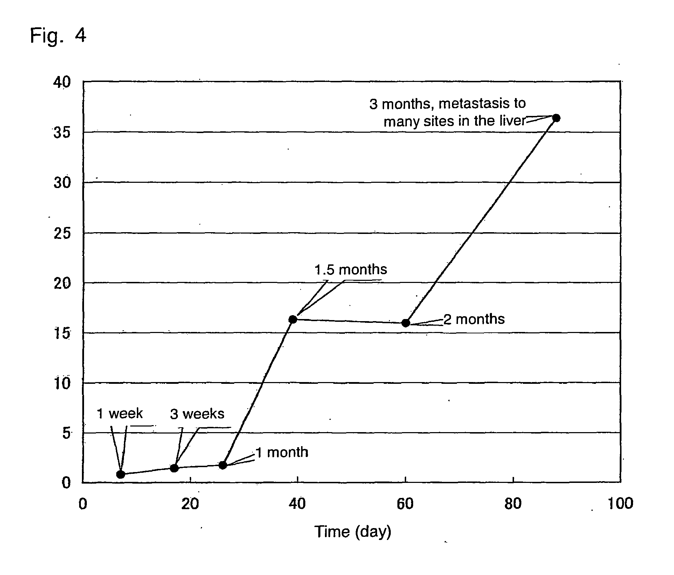

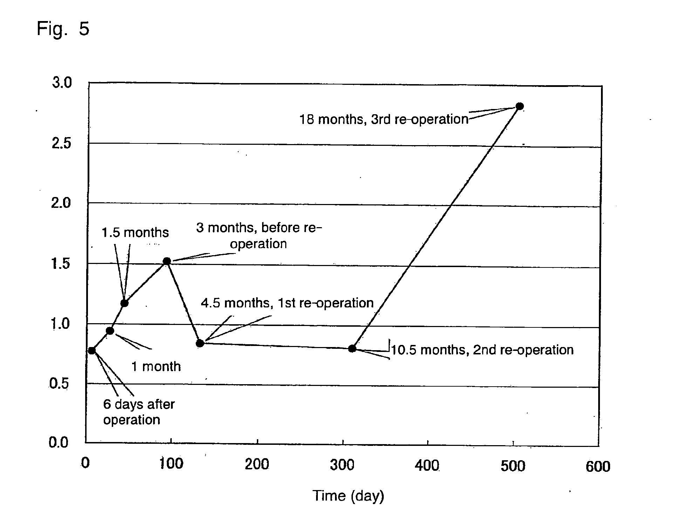

Serum cystatin SN levels of five large intestinal cancer patients after excision of cancer were monitored over time. Representative results are shown in Figs. 4 and 5. Fig. 4 shows a case of metastasis to the liver after surgery, in which serum cystatin SN is increased with time period after surgery. The result indicates that metastasis after surgery can be monitored on the basis of serum cystatin SN level. Fig. 5 shows a case of repeated recurrence after surgery with operating four surgeries. In Fig. 5, the serum cystatin SN level drops slightly after each surgical operation, but increases again after recurrence. Therefore, recurrence of cancer can be predicted through monitoring cystatin SN level after surgery.

### Example 9 (Comparison between ELISA systems)

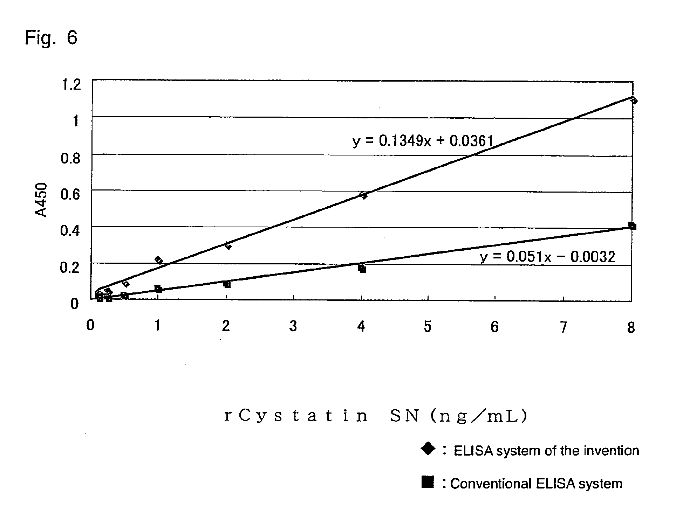

A standard curve obtained from a sandwich ELISA system employing PPMX0201 as an immobilized antibody and a polyclonal antibody as a biotin-labeled antibody (conventional) was compared with that obtained from an ELISA system employed in the invention employing FERM BP-10451 as an immobilized antibody and FERM BP-10452 as a biotin-labeled antibody. As shown in Fig. 6, the curve obtained in the conventional system is represented by the equation: y=0.051x-0.0032, whereas the assay system employed in the present invention is represented by the equation: y=0.1349x+0.0361, indicating that an assay system with high sensitivity has been developed successfully.

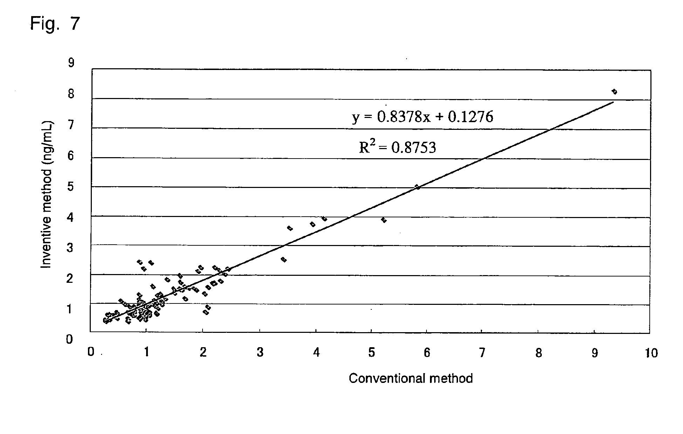

Correlation between the conventional ELISA system and the ELISA system employed in the present invention was calculated through plotting the serum cystatin SN levels of the patients. The correlation is represented by the equation: y=0.8378x+0.1276, with R²=0.8753. Thus, good correlation was observed, although there were some exceptional data in a range of low concentration (Fig. 7).

### Example 10 (Detection of serum cystatin SN in large intestinal cancer patients, large intestinal polyp patients, and healthy subjects)

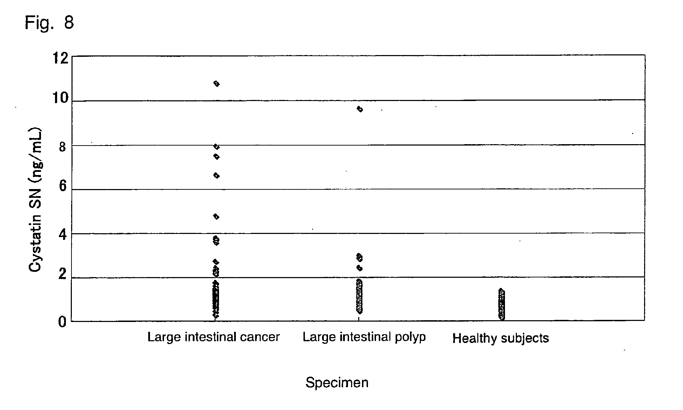

By means of the ELISA system employed in the invention, serum cystatin SN was detected in 58 large intestinal cancer patients, 37 large intestinal polyp patients, and 70 healthy subjects. The average serum cystatin SN levels of the cancer patients, polyp patients, and healthy subjects were found to be 1.93 (SD = 1.98), 1.43 (SD = 1.52), and 0.69 (SD = 0.28), respectively. As shown in Fig. 8, not only the positive rate of large intestinal cancer was increased, but also polyp conditions including those in precancerous state were enabled to be diagnosed successfully with the high-sensitivity assay system.

## Claims

1. A drug for diagnosing large intestinal cancer and/or polyp, wherein the drug comprises an anti-cystatin SN antibody.

2. A drug for monitoring recurrence and metastasis after surgery of large intestinal cancer, wherein the drug comprises an anti-cystatin SN antibody.

3. The drug for diagnosing according to claim 1, which comprises the anti-cystatin SN antibody immobilized on a support and the anti-cystatin SN antibody labeled with a labeling substance.

4. The drug for monitoring according to claim 2, which comprises the anti-cystatin SN antibody immobilized on a support and the anti-cystatin SN antibody labeled with a labeling substance.

5. A method for diagnosing large intestinal cancer and/or polyp, wherein the method comprises determining cystatin SN level in an assay sample by use of an anti-cystatin SN antibody.

6. A method for monitoring recurrence and metastasis after surgery of large intestinal cancer, wherein the method comprises determining cystatin SN level in an assay sample by use of an anti-cystatin SN antibody.

7. The method for diagnosing method according to claim 5 or the method for monitoring according to claim 6, wherein the assay sample is blood, serum, or plasma.
